(19) Europäisches Patentamt
European Patent Office
Office européen des brevets

(11) Numéro de publication : **0 477 057 A1**

(12) # DEMANDE DE BREVET EUROPEEN

(21) Numéro de dépôt : **91402345.2**

(51) Int. Cl.[5] : **A61K 7/06**

(22) Date de dépôt : **02.09.91**

(30) Priorité : **05.09.90 FR 9011034**

(43) Date de publication de la demande :
**25.03.92 Bulletin 92/13**

(84) Etats contractants désignés :
**AT BE CH DE DK ES FR GB GR IT LI NL SE**

(71) Demandeur : **L'OREAL**
**14, Rue Royale**
**F-75008 Paris (FR)**

(72) Inventeur : **Sebag, Henri**
**26, rue Erlanger**
**F-75016 Paris (FR)**
Inventeur : **Hansenne, Isabelle**
**156-158 Rue Legendre**
**F-75017 Paris (FR)**

(74) Mandataire : **Casalonga, Axel et al**
**BUREAU D.A. CASALONGA - JOSSE**
**Morassistrasse 8**
**W-8000 München 5 (DE)**

(54) **Composition de lavage et/ou de traitement cosmétique avec rinçage des fibres kératiniques, à séchage rapide, à base de composés cationiques fluorés et d'alkylpolyglycosides, et procédés mettant en oeuvre ces compositions.**

(57) Composition de lavage et/ou de traitement cosmétique avec rinçage des fibres kératiniques et en particulier des cheveux humains, à séchage rapide, contenant dans un véhicule aqueux cosmétiquement acceptable, au moins un tensio-actif non ionique du type alkylpolyglycoside et au moins un composé fluoré cationique de formule :

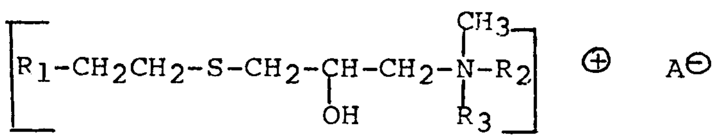

(I)

dans laquelle :

$R_1$ désigne un ou des mélanges de groupement(s) perfluoroalkyle(s) en $C_4$-$C_{16}$ ;

$R_2$ et $R_3$ désignent, indépendamment l'un de l'autre, le groupement méthyle ou éthyle ;

$A^\ominus$ désigne les ions chlorure, méthylsulfate ou éthylsulfate.

Cette composition est stable et présente de bonnes propriétés de détergence et de moussage.

EP 0 477 057 A1

La présente invention concerne des compositions de lavage et/ou de traitement cosmétique des fibres kératiniques, en particulier des cheveux humains, contenant au moins un composé cationique fluoré et un alkylpolyglycoside, ainsi que les procédés mettant en oeuvre ces compositions.

Certains composés cationiques fluorés sont utilisés en cosmétique pour leurs propriétés tensioactives dans diverses compositions détergentes et notamment dans des shampooings capillaires. De tels composés ont été décrits dans la demande de brevet européen n°0 002 004.

D'autres composés cationiques fluorés ont également été décrits dans le brevet US 4 183 367.

Les compositions de shampooing or de rinçage de l'état de la technique renferment généralement des agents tensio-actifs ayant des propriétés détergentes et moussantes. Les agents tensio-actifs non ioniques sont couramment utilisés en raison de leur faible agressivité vis-à-vis des fibres kératiniques.

Au cours de ses recherches, la demanderesse a constaté que les composés fluorés cationiques connus utilisés dans les compositions de shampooings ou de rinçage des cheveux, étaient incompatibles avec certains tensio-acifs non ioniques et conduisaient à des compositions instables dans le temps et/ou présentant des propriétés de détergence et un pouvoir moussant insuffisants pour leur utilisation cosmétique.

La demanderesse a découvert d'une manière surprenante que l'association de composés fluorés cationique particuliers tels que définis ci-après avec des agents tensio-actifs non-ioniques de la famille des alkylpolyglycosides, permettait d'obtenir des compositions de lavage et/ou de traitement avec rinçage des cheveux, à séchage rapide, stables et présentant de remarquables propriétés de détergence et de moussage.

La présente invention a donc pour objet une composition de lavage et/ou de traitement cosmétique avec rinçage des fibres kératiniques, en particulier des cheveux, à séchage rapide, renfermant un composé fluoré cationique associé à un composé alkylpolyglycoside, dans un véhicule aqueux.

Un autre objet de l'invention consiste en un procédé de lavage et/ou de traitement cosmétique avec rinçage des cheveux, mettant en oeuvre ces compositions.

On appelle "traitement cosmétique" un traitement visant à améliorer les propriétés cosmétiques des cheveux.

D'autres objets de l'invention apparaîtront à la lumière de la description.

Les compositions de lavage et/ou de traitement avec rinçage des fibres kératiniques, et en particulier des cheveux, conformes à l'invention, sont caractérisées par le fait qu'elles contiennent dans un milieu aqueux cosmétiquement acceptables :

– au moins un alkylpolyglycoside;

– au moins un composé fluoré cationique,

répondant à la formule suivante :

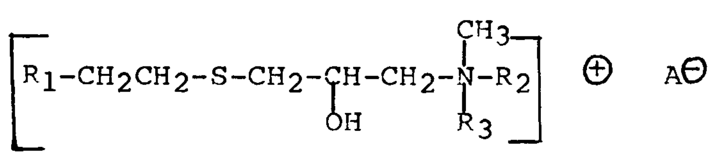

(I)

dans laquelle :

$R_1$ désigne un ou des mélanges de groupement(s) perfluoroalkyle(s) en $C_4$-$C_{16}$;

$R_2$ et $R_3$ désignent, indépendamment l'un de l'autre, le groupement méthyle ou éthyle;

A⊖ désigne les ions chlorure, méthylsulfate ou éthylsulfate.

Les alkylpolyglycosides utilisés conformément à la présente invention, répondent en particulier à la formule (II) :

$$R\text{-}(C_6H_{10}O_5)_xH \qquad (II)$$

ou encore correspondent à la formule développée (III) :

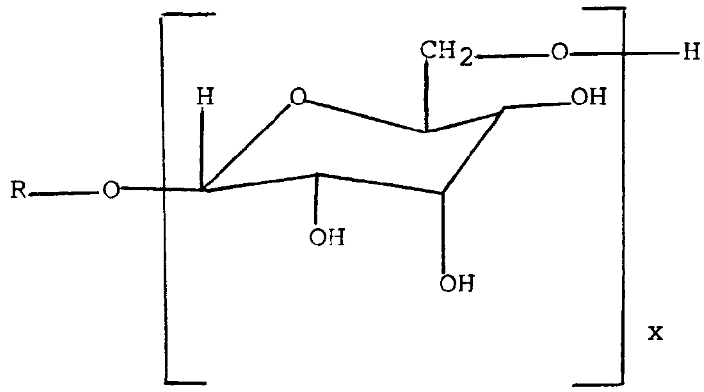

(III)

dans lesquelles :

x est un nombre entier de 1 à 15;

R désigne un radical or un mélange de radicaux alkyle saturé ou insaturé, à chaîne droite ou ramifiée en $C_8$-$C_{24}$.

De tels produits sont vendus, notamment par la Société HORIZON CHEMICAL sous la dénomination APG (APG 300, APG 350, APG 500, APG 550, APG 625, APG Base 10-12); par la Société HENKEL sous la dénomination MERGITAL CG 60; par la Société ROHM et HAAS sous la dénomination TRITON CG 110, TRITON CG 312, ORAMIX CG 110, ORAMIX NS 10; par la Société BASF sous la dénomination LUTENSOL GD 70.

Les composés alkylpolyglycosides utilisés préférentiellement, selon la présente invention, sont les composés de formules (II) ou (III) tels que définis ci-dessus, dans lesquels R est un radical alkyle saturé en $C_8$ à $C_{11}$ et x est compris entre 1 et 10; ils sont vendus en particulier sous la dénomination APG 300 par la Société HORIZON CHEMICAL sous forme de solution aqueuse à 50% de matière active.

Les composés alkylpolyglycosides sont de préférence utilisés dans les compositions selon l'invention, dans des quantités comprises entre 0,1 et 50% en poids par rapport au poids total des compositions.

Les composés cationiques fluorés de formule (I) utilisés selon la présente invention, sont connus en eux-mêmes. Ils sont décrits dans la demande de brevet européen EP-002 004.

Un composé fluoré cationique de formule (I) particulièrement préféré selon la présente invention, est celui répondant à la structure suivante :

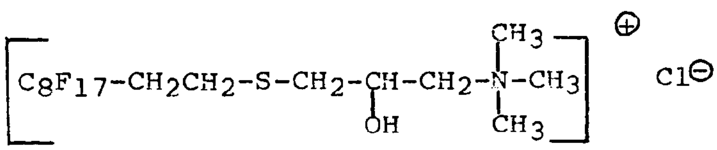

Les composés fluorés cationiques de formule (I) sont de préférence utilisés dans des quantités comprises entre 0,05 et 5% en poids par rapport au poids total des compositions.

Lorsque la composition est sous la forme d'une composition destinée au lavage et au traitement cosmétique des cheveux, notamment sous la forme d'un shampooing, elle renferme de 5 à 50% en poids d'alkylpolyglycosides du poids total de la composition.

Lorsque la composition est sous la forme d'une composition destinée au traitement cosmétique des cheveux, notamment sous la forme d'un après-shampooing ou d'une composition de rinçage, elle renferme de 0,1 à 50% en poids d'alkylpolyglycosides du poids total de la composition.

Les compositions conformes à la présente invention présentent un pH généralement compris entre 2 et 9 et plus particulièrement entre 4,5 et 7,5.

Le véhicule aqueux peut être constitué par de l'eau ou par un mélange d'eau et d'un solvant cosmétiquement acceptable choisi parmi les alcools inférieurs en $C_1$-$C_4$, comme l'éthanol, l'isopropanol, le n-butanol; les alkylèneglycols comme l'éthylène glycol; les éthers de glycol. Lorque le véhicule est constitué par un mélange d'eau et de solvant, la quantité de solvant présent est égale ou inférieure à 10% en poids et de préférence elle est égale ou inférieure à 5% en poids du poids total de la composition.

Les compositions selon l'invention se présentent sous forme de liquide, de liquide épaissi, de gel, de crème, de mousse aérosol ou de spray et peuvent contenir les adjuvants habituellement utilisés dans les compositions cosmétiques de ce type.

Elles peuvent également contenir en plus des agents de traitement ayant pour effet d'améliorer les propriétés cosmétiques des cheveux sans altérer la stabilité de la composition ainsi que des adjuvants et plus particulièrement des parfums, des conservateurs, des séquestrants, des stabilisateurs de mousse, des agents propulseurs, des colorants, des agents acidifiants ou alcalinisants.

Les compositions pour le lavage et le traitement cosmétique des cheveux sont utilisées notamment comme composition de shampooing capillaire.

Les compositions pour le traitement cosmétique des cheveux sont utilisées notamment comme composition "après-shampooing" ou composition de rinçage des cheveux à séchage rapide. Elles sont appliquées sur les cheveux humides ou secs dans des quantités efficaces pour les laver et/ou les traiter, cette application étant suivie d'un rinçage à l'eau.

Les examples qui suivent servent à illustrer la présente invention sans toutefois présenter un caractère limitatif.

## EXEMPLE 1

On prépare un shamppoing de composition suivante :

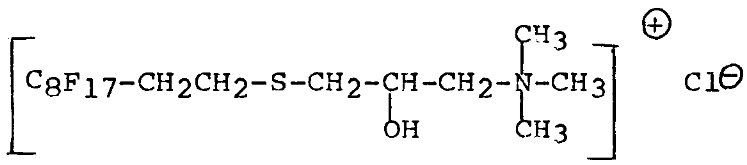 2 g

- Alkylpolyglycoside vendu par la Société HORIZON CHEMICAL sous la dénomination APG 300 en solution à 50% de matière active (MA) — 15 g MA
- Agent séquestrant, conservateur, parfum — qs
- Triéthanolamine — qs pH = 6
- Eau — qsp 100 g

On obtient un shampooing très détergent et moussant qui accélère le séchage des cheveux humides. MA = matière active.

## EXEMPLE 2

On prépare un shampooing de composition suivante :

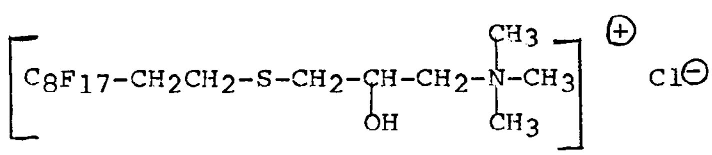 3 g MA

- Alkylpolyglycoside vendu par la Société HORIZON CHEMICAL sous la dénomination APG 550 en solution à 50% de matière active (MA) — 25 g MA
- Agent séquestrant, conservateur, parfum — qs
- Triéthanolamine — qs pH = 6
- Eau — qsp 100 g

Le shampooing très détergent et moussant accélère le séchage des cheveux humides.

## EXEMPLE 3

On prépare un shampooing de composition suivante :

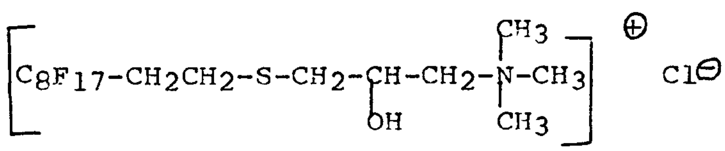

4 g MA

- Alkylpolyglycoside vendu par la Société BASF sous la dénomination LUTENSOL GD 70 en solution à 70% de matière active (MA) — 35 g MA
- Agent séquestrant, conservateur, parfum — qs
- Triéthanolamine — qs pH = 6
- Eau — qsp 100 g

Le shampooing très détergent et moussant accélère le séchage des cheveux humides.

## EXEMPLE 4

On prépare un shampooing de composition suivante :
Composé de formule :

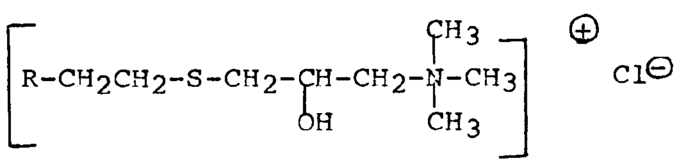

dans laquelle R est un mélange de radicaux perfluoroalkyle en $C_4$-$C_{16}$ vendu sous la dénomination LODYNE S106B par la Société CIBA-GEIGY à 30% de MA — 2 g MA

- Alkylpolyglycoside vendu par la Société HORIZON CHEMICAL sous la dénomination APG 300 en solution à 50% de matière active (MA) — 15 g MA
- Agent séquestrant, conservateur, parfum — qs
- Triéthanolamine — qs pH = 6
- Eau — qsp 100 g

Le shampooing très détergent et moussant accélère le séchage des cheveux humides.

EXEMPLE 5

On prépare un shampooing de composition suivante :
Composé de formule :

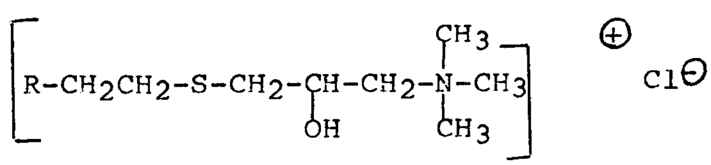

dans laquelle R est un mélange de radicaux perfluoroalkyle en $C_4$-$C_{16}$ vendu sous la dénomination LODYNE S106B par la Société CIBA-GEIGY à 30% de MA — 1 g MA

- Alkylpolyglycoside vendu par la Société HORIZON CHEMICAL sous la dénomination APG 550 en solution à 50% de matière active (MA) — 10 g MA
- Agent séquestrant, conservateur, parfum — qs
- Triéthanolamine — qs pH = 6
- Eau — qsp 100 g

Le shampooing très détergent et moussant accélère le séchage des cheveux humides.

EXEMPLE 6

On prépare un après-shampooing de composition suivante :

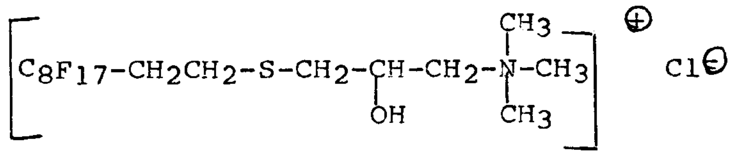

1 g MA

- Alkylpolyglycoside vendu par la Société HORIZON CHEMICAL sous la dénomination APG 300 en solution à 50% de matière active (MA) — 2 g MA
- Chlorure de distéaryldiméthylammonium — 1 g
- Agent séquestrant, conservateur parfum qs
- pH spontané = 5
- Eau qsp 100 g

Cet après-shampooing accélère le séchage des cheveux humides.

## Revendications

1. Composition de lavage et/ou de traitement cosmétique avec rinçage des fibres kératiniques, en particulier des cheveux, à séchage rapide, caractérisée par le fait qu'elle contient dans un véhicule aqueux cosmétiquement acceptable, au moins un composé alkylpolyglycoside et au moins un composé cationique fluoré de formule :

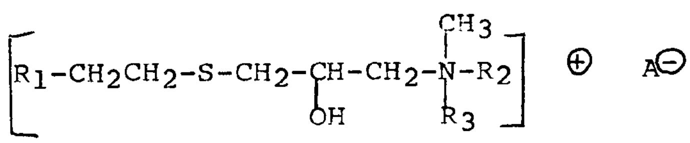

(I)

dans laquelle :
$R_1$ désigne un groupement ou un mélange de groupements perfluoroalkyles en $C_4$-$C_{16}$ ;
$R_2$ et $R_3$ désignent, indépendamment l'un de l'autre, le groupement méthyle or éthyle;
$A^{\ominus}$ désigne les ions chlorure, méthylsulfate or éthylsulfate.

2. Composition selon la revendication 1, caractérisée par le fait que l'alkylpolyglycoside est choisi parmi :
(i) ceux répondant à la formule :

$$R\text{-}(C_6H_{10}O_5)_xH \qquad (II)$$

ou (ii) ceux répondant à la formule :

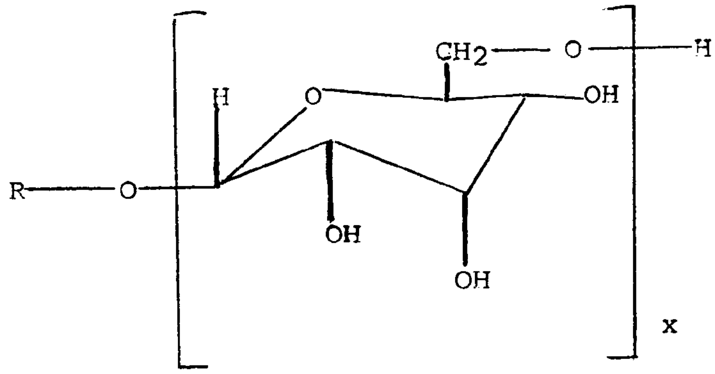

(III)

dans lesquelles :

R désigne un radical ou un mélange de radicaux alkyle saturé ou insaturé, à chaîne droite ou ramifiée en $C_8$-$C_{24}$;

x est un nombre entier de 1 à 15.

**3.** Composition selon la revendication 2, dans laquelle R est un radical aliphatique saturé en $C_8$ à $C_{11}$.

**4.** Composition selon l'une quelconque des revendications 1 à 3, destinée au lavage et au traitement cosmétique des cheveux, à séchage rapide, caractérisée par le fait qu'elle contient de 5 à 50% en poids d'alkylpolyglycosides du poids total de la composition.

**5.** Composition selon l'une quelconque des revendications 1 à 3, destinée au traitement cosmétique des cheveux, à séchage rapide, caractérisée par le fait qu'elle contient de 0,1 à 50% en poids d'alkylpolyglycosides du poids total de la composition.

**6.** Composition selon l'une quelconque des revendications 1 à 5, caractérisée par le fait que le composé fluoré cationique répond à la formule suivante :

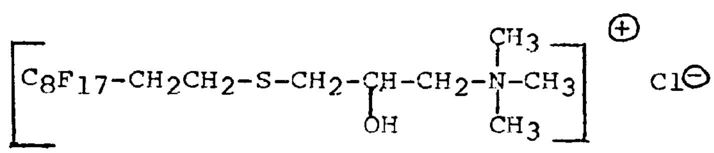

**7.** Composition selon l'une quelconque des revendications 1 à 6, caractérisée par le fait que le composé fluoré cationique est présent dans une quantité de 0,05 à 5% en poids par rapport au poids total de la composition.

**8.** Composition selon l'une quelconque des revendications 1 à 7, caractérisée par le fait que le véhicule aqueux est constitué par de l'eau ou un mélange d'eau et d'un solvant cosmétiquement acceptable choisi parmi les alcools inférieurs en $C_1$-$C_4$, les alkylène glycols et les éthers de glycol.

**9.** Composition selon la revendication 8, caractérisée par le fait que le véhicule aqueux est constitué par un mélange d'eau et de solvant, la quantité de solvant présent est égale ou inférieure à 10% en poids et de préférence égale ou inférieure à 5% en poids du poids total de la composition.

**10.** Composition selon l'une quelconque des revendications 1 à 9, caractérisée par le fait qu'elle se présente sous forme de liquide, de liquide épaissi, de gel, de crème, de mousse aérosol ou de spray.

**11.** Composition selon l'une quelconque des revendications 1 à 10, caractérisée par le fait que le pH est compris entre 2 et 9.

**12.** Composition selon l'une quelconque des revendications 1 à 11, caractérisée par le fait qu'elle contient également des agents de traitement pour les cheveux qui améliorent les propriétés cosmétiques des cheveux sans altérer la stabilité de la composition et/ou des adjuvants choisis parmi les parfums, les agents séquestrants, les agents conservateurs, les épaississants, les adoucissants, les stabilisateurs de mousses, les agents propulseurs, les agents acidifiants, les agents alcalinisants.

**13.** Procédé de lavage et de traitement cosmétique des cheveux, à séchage rapide des cheveux, caractérisé par le fait que l'on applique sur les cheveux humides ou secs une quantité efficace pour le lavage et le traitement cosmétique des cheveux d'une composition selon l'une quelconque des revendications 1 à 4 et 6 à 11, cette application étant suivie d'un rinçage à l'eau.

**14.** Procédé de traitement cosmétique des cheveux, caractérisé par le fait que l'on applique sur des cheveux humides une quantité efficace pour le traitement cosmétique des cheveux d'une composition selon l'une quelconque des revendications 1 à 3 et 5 à 12, cette application étant suivie d'un rinçage.

Office européen des brevets

# RAPPORT DE RECHERCHE EUROPEENNE

Numero de la demande

EP 91 40 2345

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | EP-A-0 002 004 (CIBA-GEIGY AG.)<br>* Document complet *<br>--- | 1,8 | A 61 K 7/06 |
| A | GB-A-2 185 992 (COLGATE-PALMOLIVE CO.)<br>* Page 1, ligne 58 - page 2, ligne 43; exemple 1 *<br>--- | 1-3 | |
| A | EP-A-0 357 484 (L'OREAL)<br>* Page 3, ligne 46 - page 4, ligne 20; revendications 1,4 *<br>----- | 1-3 | |

DOMAINES TECHNIQUES RECHERCHES (Int. Cl.5)

A 61 K
C 11 D

Le présent rapport a été établi pour toutes les revendications

| Lieu de la recherche | Date d'achèvement de la recherche | Examinateur |
|---|---|---|
| LA HAYE | 13-12-1991 | COUCKUYT P.J.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)